# EUROPEAN PATENT APPLICATION

(11) **EP 4 277 046 A1**
(43) Date of publication of application: **15.11.2023**
(21) Application number: 23172508.6
(22) Date of filing: 10.05.2023
(51) Int. Cl.: H01R 24/86, A61B 18/14, H01R 4/02

(54) **HIGH VOLTAGE IRREVERSABLE ELECTROPORATION (IRE) CONNECTOR**

(30) Priority: 11.05.2022 US 202217741537
(71) Applicant: BIOSENSE WEBSTER (ISRAEL) LTD., 2066717 Yokneam (IL)
(72) Inventor: BEN SHOSHAN, Sharona, 2066717 Yokneam (IL); WISEMAN, Yaron, 2066717 Yokneam (IL)
(74) Representative: Carpmaels & Ransford LLP

(57) **Abstract**

Electrical connectors for carrying high voltages for Irreversible Electroporation (IRE) include a main connector which supports electrical contacts at two or more different elevations on the connector body.

## Description

### TECHNICAL FIELD

The present disclosure relates generally to electrical connectors for medical applications, and particularly to high voltage electrical connector assemblies for catheters used in Irreversible Electroporation (IRE) procedures.

### BACKGROUND

Irreversible electroporation (IRE) catheters include an array of electrodes for creating a desired field. Typically, each electrode is associated with a channel, so that it can be individually controlled. The electrodes of the IRE catheter are configured to receive high voltages from the power or IRE generator, typically within a console.

### SUMMARY OF THE DISCLOSURE

The disclosed subject matter provides a connector assembly, formed of first and second connectors, with electrical contacts and channels arranged to support the large number of channels needed to carry high voltages and high voltage signals used in irreversible electroporation (IRE) applications. The first or main carrier provides a leveled structure for carrying electrical contacts at each of the levels, including at least two levels for carrying high voltage electrical contacts. This arrangement maximizes the real estate available on the main connector, for carrying high voltage electrical contacts, while keeping the diameter of the main connector at its present size, and therefore, not increasing the diameter of the connector assembly.

### BRIEF DESCRIPTION OF THE DRAWINGS

The present disclosure will be more fully understood from the following detailed description of the examples thereof, taken together with the accompanying drawings. In the drawings, identical or similar structures, elements or parts that appear in more than one figure are generally labeled with the same numeral and/or character in all the drawings in which they appear, and a numeral and/or character labeling an icon representing a given feature in a drawing may be used to reference the given feature. Dimensions of components and features shown in the drawings are chosen for convenience and clarity of presentation and are not necessarily shown to scale. In the drawings:
Fig. 1 is a perspective view of a connector assembly formed of joined connectors, in accordance with an example of the present disclosure;
Fig. 2 is a perspective view of the connector assembly of Fig. 1 with the connectors separated;
Fig. 3A is a front perspective view of the main connector of the connector assembly of Figs. 1 and 2;
Fig. 3B is a front perspective view of the main connector and the electrical contacts, with a flat printed circuit attached in detail;
Fig. 4 is a rear view of the main connector of the connector assembly of Figs. 1 and 2; and
Fig. 5 is a front perspective view of the mating connector of the connector assembly of Figs. 1 and 2.

### DETAILED DESCRIPTION OF EXAMPLES

### OVERVIEW

Irreversible electroporation (IRE) catheters have multiple high voltage channels that need to fit into a connector for connecting with a mating connector associated with an IRE generator. The channels are soldered to the connector in a manual process, with the channels terminating in contacts, i.e., electrical contacts. After soldering, the residual soldering flux is removed in a cleaning process. Due to the relatively small footprint of the connector, even a small amount of soldering flux may cause a short between the contacts of the channels (lines). As the number of electrical contacts and corresponding channels increases, a distance or pitch between each of the contacts and corresponding channels, for example, of approximately 1 mm distance for each 1 KV, needs to be maintained for safety.

Should a connector assembly suitable for 2 KV, be desired with the multiple channels (lines) spread apart with the spacing of approximately 1 mm distance for each 1 KV between each of the channels, will result in a very large connector assembly that is not convenient or practical for use with a handheld catheter, and in particular, an IRE catheter. Furthermore, soldering so many lines in a small footprint is difficult, inconvenient, and therefore also costly to manufacture.

The present disclosure provides connectors which can handle the present and ever-increasing number of electrical contacts (for example, including electrodes) and corresponding channels, for carrying high voltages of IRE, of approximately 2-4 KV.

The present disclosure provides a connector structure which supports electrical contacts at two or more different elevations. This structure maximizes real estate for electrical contacts and corresponding channels, without increasing diameter of the connector, while maintaining the necessary distance between each of the electrical contacts and corresponding channels, to avoid electrical short circuiting and voltage creepage between channels. The resultant connectors allow for the safe operation of high voltage IRE pulses to be transmitted from an IRE generator to the catheter, through the disclosed connectors.

### SYSTEM DESCRIPTION

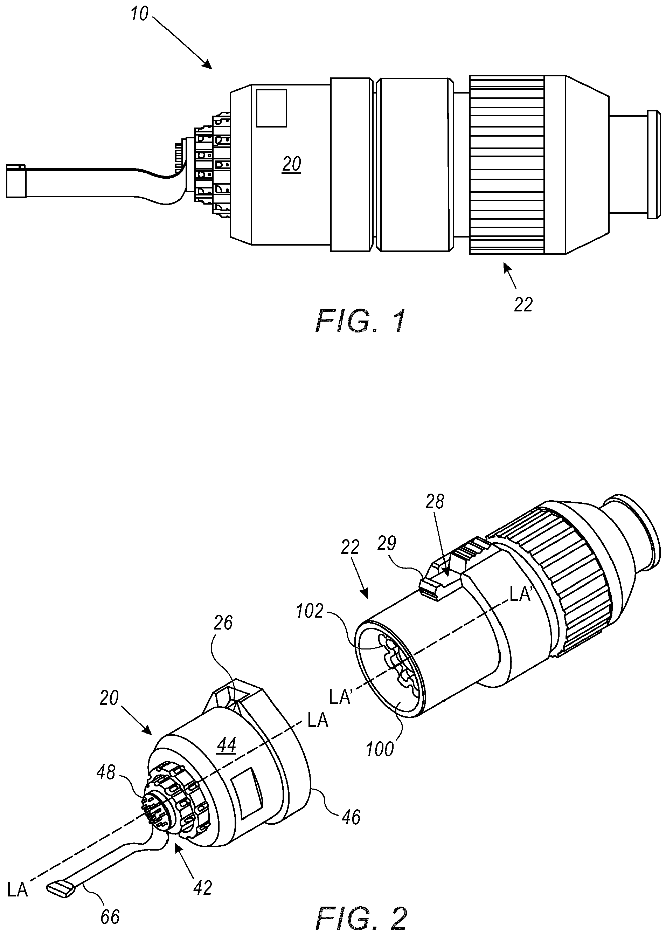

Reference is initially made to Figs 1 and 2, which show a connector assembly 10 formed of a first connector or main connector 20, electrically coupled, and mechanically coupled to a second connector or mating connector 22. The main connector 20 communicates with an IRE catheter, while the mating connector 22, communicates with an IRE generator. The main connector 20, when electrically coupled with the mating connector 22 is able to carry high voltages needed for IRE, of 2-4KV, as well as low voltages of approximately 1 Kilovolt (KV) or less, while maintaining the electrical contacts and corresponding channels at a safe (predetermined) distance from each other. By maintaining the electrical contacts and corresponding channels at safe distances or pitches apart from each other, potential short circuiting from physical contact and/or creepage (the breakthrough of voltage between two contacts or channels at short distances, but not in physical contact, from each other), is avoided. This allows the voltages and signals to be transmitted from the IRE generator to the catheter, and ultimately to the patient safely, avoiding an IRE system malfunctions, which could harm the patient.

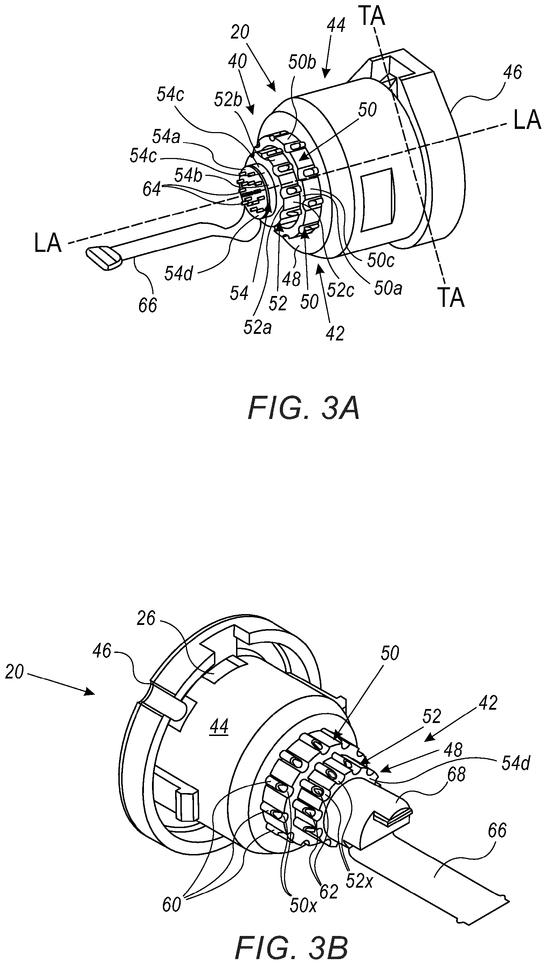

The main connector 20 includes apertures 26, which receive flexible arms 28, having shoulders 29, which are moveable in and out of the apertures 26, when coupling or connection and decoupling or disconnection of the connectors 20, 22, from each other, is desired. Turning also to Figs. 3A and 3B, the main connector 20 includes a body 40, formed by a head 42 extending from a base 44. The head 42 extends outward from the base 44 in a longitudinal dimension (along a longitudinal axis LA). As used in this document, the term "longitudinal" refers to the direction passing between the proximal 46 and distal 48 ends of the body 40 of the main connector 20. The head 42 and base 44 are, for example, concentric and coaxial (about the longitudinal axis LA) with each other. A transverse axis TA is oriented perpendicular to the longitudinal axis LA and represents a transverse direction with respect to the body 40.

The body 40 is typically formed of an insulating material, such as a polymer. The insulation is, for example, up to approximately 6KV, to prevent creepage between each of the electrical contacts and/or channels (and components thereof, such as wires).

The body 40, at the head 42, for example, includes a plurality of concentric and coaxial platforms 50 (e.g., first), 52 (e.g., second), 54 (e.g., third), extending telescopically distally, toward the distal end 48 of the body 40. For example, three platforms 50, 52, 54 are shown, although other numbers of plural platforms are permissible. The platforms 50, 52, 54 are narrower moving distally. Each platform 50, 52, 54, is, for example, cylindrical in shape, with a circular or rounded cross section, extending transversely to a periphery 50a, 52a, 54a. The cylindrical portion 50b, 52b, 54b terminates in a peripheral surface 50c, 52c, 54c, each surface, for example, running substantially parallel to the longitudinal axis LA. Each successive platform 50, 52, 54, when moving distally along the head 42, is, for example, narrower (e.g., in diameter in the transverse direction) than the previous platform, and fits within the periphery of the previous platform (e.g., the previous platform proximal to the instant platform in the longitudinal direction).

For the widest two platforms 50, 52 electrical contacts 60, 62, are, for example in cut outs 50x, 52x (e.g., rounded in shape), extending into the peripheral surfaces 50c, 52c, and for example, arranged as rings (e.g., circular in shape) of contacts 60, 62, such that the electrical contacts 60 of the first platform 50, which form an outer ring, are at a different elevation, e.g., lower, elevation than the electrical contacts 62 of the second platform 52, which form an inner ring. The rings of electrical contacts 60, 62, are for example, concentric and coaxial about the longitudinal axis LA.

The electrical contacts 60, 62 include, for example, solder cups, for example, of gold over nickel, spaced or pitched apart from each other at a predetermined distance to eliminate physical contact and/or creeping. The space distance or pitch is for example, at least 4 mm, allowing the contacts 60, 62 and corresponding channels to carry high voltages, up to approximately 4KV loads. For example, both platforms 50, 52, support twelve high voltage electrical contacts 60, 62, in the form of solder cups.

The solder cups are joined to wires (not shown), which connect to electrical contacts of the IRE catheter. The spacing distance between each of the electrical contacts 60 of the first platform 50 and the electrical contacts 62 of the second platform 52 may be the same or different, provided that the minimum spacing distance, e.g., of at least 4 mm, is maintained.

The narrowest, and distal-most platform 54 supports electrical contacts 64, for example, in the form of pins. The pins protrude from the platform surface 54d, and are designed to carry low voltages (low voltage signals), e.g., 1 KV or less. Accordingly, the space distance or pitch between the pins (forming the electrical contacts 64) is at least 1mm. The pins (forming the electrical contacts 64) are, for example, made of gold over nickel. For example, there are sixteen pins.

Also, for example, the electrical contacts 64 communicate with a flat printed circuit (FPC) 66. The FPC 66 is of a flexible (non-rigid) material and communicates with, for example, connects to, a printed circuit board. The FPC 66 may be, for example, a Molex^{®} (16 Position) PiN 5051101692 FPC. A cap 68 typically covers the electrical contacts 64 on the distal most platform 54, as shown in Fig. 3B.

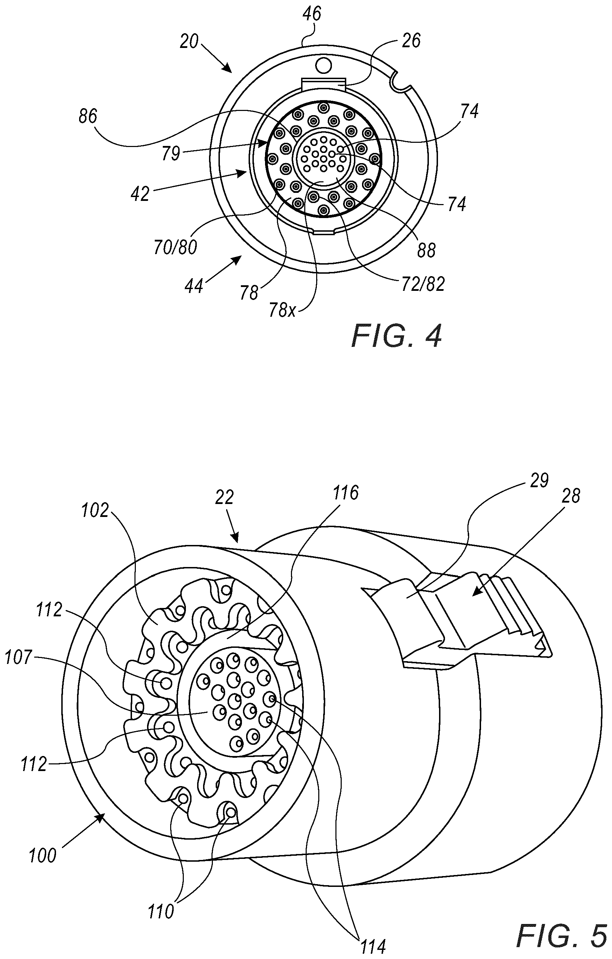

As shown in Fig. 4, at the proximal end 46 of the body 40 of the main connector 20, at the base 44, are electrical contacts 70, 72, 74, corresponding to the respective electrical contacts 60, 62, 64 at the head 42 of the body 40. The electrical contacts 70, 72, 74, are, for example, pins, of an electrically conductive material, including electrically conductive metals, extending outward from the base surface 78, inside a cylinder segment 79.

Electrical contacts 70, 72 are high voltage electrical contacts, and communicate with electrical contacts 60, 62, of the respective first platform 50 and the second platform 52, via channels (not shown). The channels (not shown), are, for example, formed of electrically conductive wires (not shown), and extend through the body 40 between the respective electrical contacts 60, 62, 70, 72, in an aligned manner. For example, the separation distance or pitch between each of the channels is at least approximately 4 mm, to accommodate the high voltage, e.g., high voltage signals.

The electrical contacts 70 at the base 44 which correspond to the electrical contacts 60 of the first (first or lower elevation-outer ring) platform 50, are arranged, for example, as an outer ring 80 (e.g., circular in shape). Similarly, for example, the electrical contacts 72 at the base 44 which correspond to the electrical contacts 62 of the second (second of higher elevation-inner ring) platform 52, are arranged as an inner ring 82 (e.g., circular in shape), within the outer ring 80. For example, the inner ring 80 and outer ring 82 are circular in shape, but may be other shapes, provided the separation distance or pitch between each of the electrical contacts 70, 72 is at least approximately 4 mm, to accommodate the high voltage, e.g., high voltage signals.

Additionally, both the outer ring 80 and the inner ring 82 each include twelve electrical contacts, corresponding to the electrical contacts at the lower elevation platform 50 (e.g., the electrical contacts 60 arranged as an outer ring along the peripheral surface 50c of the of platform 50), and the higher elevation platform 52 (e.g., the electrical contacts 62 arranged as an inner ring along the peripheral surface 52c of the of platform 52), respectively. The respective channels align in accordance with the position of the electrical contacts 60, 62, 70, 72 on the respective outer ring of the platform 50 and the outer ring 80, and, the inner ring of the platform 52 and the inner ring 82.

At the base surface 78, within the inner ring 82 is an area 79 where electrical contacts 74, for example, pins, are arranged in a pattern identical or similar the pin arrangement of the electrical contacts 64, as arranged on the distal-most platform 54. Channels, formed of wires extend through the body 40, between aligned and paired electrical contacts 64, 74. These electrical contacts 64, 74 and corresponding channels handle low voltages, for example, of approximately 1 KV or less and as such are spaced or pitched apart from each other at a sufficient distance, to avoid contact and/or creepage, for example, at least 1 mm. Additionally, the low voltage electrical contacts 74 are separated from the high voltage electrical contacts 70, 72, by a circular wall 86. Within the circular wall 86 is an area 88, where the electrical contacts 74 extend outward from a base surface 78x, which, for example is the main base surface 78, just inside the wall 86. The circular wall 86 is, for example, inside the inner ring 82 of high voltage electrical contacts 72.

For example, an example connector 20 with the connector body 40 supports a total of 40 channels- twentyfour high voltage channels between paired electrical contacts 60, 70, 62, 72, and sixteen low voltage channels between paired electrical contacts 64, 74.

Fig. 5 shows the mating or second connector 22. The mating connector 22 is configured correspondingly with respect to the base 44 and base surfaces 78, 78x to receive and engage the electrical contacts 70, 72, 74 of the main connector 20, resulting in an electrical coupling between the main connector 20 and the mating connector 22, the coupled connectors forming the connector assembly 10 (Figs. 1 and 2).

The mating connector 22 includes a body segment 100, which is received in the cylinder segment 79 of the base 44 (Fig. 4). The body segment 100 houses an inner surface 102, which includes slots 110 (outer ring), 112 (inner ring), for receiving the high voltage electrical contacts 70, 72, e.g., pins, of the main connector 20, and second slots 114, for receiving the low voltage electrical contacts 74 of the main connector 20. The slots 110, 112, 114, are arranged on the inner surface 100 at positions corresponding to the positions of the electrical contacts 70, 72, 74 on the base surfaces 78, 78x of the main connector 20. Additionally, a groove 116, extending into the inner surface 102, of a shape and to a depth corresponding to the wall 86 of the main connector 20, serves to receive the wall 86 of the main connector 20, to enhance the physical engagement during the electrical coupling, between the main connector 20 and the mating connector 22. When coupled, for example, the connectors 20, 22 align along their respective longitudinal axes LA, LA' with the alignment being coaxial, as shown in Figs. 1 and 2.

While the main connector 20 is shown as having platforms 50, 52, 54, that are cylindrical with a circular cross-sectional shape, the cylinders may also be of other cross-sectional shapes, including rounded shapes such as oval, as well as rectangular, square, and triangular shapes, and combinations thereof. Similarly, the proximal 46 of the main connector 20 may be of other shapes, also for example, of other cross-sectional shapes, including rounded shapes such as oval, as well as rectangular, square, and triangular shapes, and combinations thereof. The mating connector 22 would be of a corresponding same or similar shape to that of the proximal end 46 of the main connector 20, correspondingly dimensioned therewith, to electrically couple with the main connector 20, as well as physically couple with the main connector, at the proximal end 46 of the main connector 20.

Although the examples described herein mainly address electrical connectors for IRE systems, consoles and catheters, the methods and systems described herein can also be used in other applications, such as in electrical connectors and connector assemblies which carry high voltages, for example, up to approximately 4 KV, as well as those which carry both high and low voltages, for example, a high voltage of up to approximately 4KV and a low voltage of up to approximately ±10V.

### EXAMPLES

### Example 1

An electrical connector assembly (10) comprising: a first connector (20) having a longitudinal axis (LA) extending between a proximal end (46) and a distal end (48) of the first connector, and a transverse axis (TA) extending substantially perpendicular to the longitudinal axis (LA). The first connector (20) comprises: a base (44); a first platform (50) extending longitudinally from the base (44) and extending transversely to a periphery (50a); a second platform (52) extending longitudinally from the first platform (50) and extending transversely to a periphery (52a), the periphery (52a) of the second platform (52) within the periphery (50a) of the first platform (50); a plurality of first electrical contacts (60) distributed along the periphery (50a) of the first platform (50); and, a plurality of second electrical contacts (62) distributed along the periphery (52a) of the second platform (52).

### Example 2

The electrical connector assembly (10) of Example 1, wherein the first connector (20) additionally comprises: a third platform (54) extending longitudinally from the second platform (52) and extending transversely to a periphery (54a), the periphery (54a) of the third platform (54) within the periphery (52a) of the second platform (52), the third platform (54) supporting a plurality of third electrical contacts (64).

### Example 3

The electrical connector (10) of any one of Example 1 or Example 2, wherein: the plurality of first electrical contacts (60) distributed along the periphery (50a) of the first platform (50) are spaced apart from each other at a first predetermined distance; and, the plurality of second electrical contacts (62) distributed along the periphery (52a) of the second platform (52) are spaced apart from each other at a second predetermined distance.

### Example 4

The electrical connector assembly (10) of any one of Example 1 to Example 3, wherein the first predetermined distance and the second predetermined distance are the same.

### Example 5

The electrical connector assembly (10) of any one of Example 1 to Example 4, wherein the first predetermined distance and the second predetermined distance are different.

### Example 6

The electrical connector assembly (10) of any one of Example 1 to Example 5, wherein the third electrical contacts (64) comprise pins, extending from surface (54d) of third platform (54) and for electrically communication with a flat printed circuit (66).

### Example 7

The electrical connector assembly (10) of any one of Example 1 to Example 6, wherein the first platform (50) and the second platform (52) are circular in a cross section along the transverse axis (TA) and are concentric, and, 1) the plurality of first electrical contacts (60) are arranged in an outer ring, and 2) the plurality of second electrical contacts (62) are arranged in an inner ring, with respect to the outer ring.

### Example 8

The electrical connector assembly (10) of any one of Example 1 to Example 7, wherein the first platform (50), the second platform (52), and the third platform (54) are circular in a cross section along the transverse axis (TA) and are concentric.

### Example 9

The electrical connector assembly (10) of any one of Example 1 to Example 8, wherein the first electrical contacts (60) and the second electrical contacts (62) comprise solder cups.

### Example 10

The electrical connector assembly (10) of any one of Example 1 to Example 9, additionally comprising: a plurality of pins (70, 72) in the base (44), each pin of the plurality of pins (70, 72) in electrical communication with a corresponding one of the first (60) or second (62) electrical contacts.

### Example 11

The electrical connector assembly (10) of any one of Example 1 to Example 10, wherein the base (44) is substantially cylindrical and is circular in cross section along the transverse axis (TA).

### Example 12

The electrical connector assembly (10) of any one of Example 1 to Example 11, wherein the pins (70) corresponding to the first electrical contacts (60) are arranged on the base (44) as a first ring (80), and, the pins (72) corresponding to the second electrical contacts (62) are arranged on the base (44) in a second ring (82), inside of the first ring (80).

### Example 13

The electrical connector assembly (10) of any one of Example 1 to Example 12, additionally comprising a plurality of pins (74) supported by the base (44) within the second ring (82), the each of the pins (74) in electrical communication with a corresponding third electrical contact (64).

### Example 14

The electrical connector assembly (10) of any one of Example 1 to Example 13, wherein the first electrical contacts (60) and the second electrical contacts (62) are for carrying high voltages of approximately at least 4 KV.

### Example 15

The electrical connector assembly (10) of any one of Example 1 to Example 14, wherein the third electrical contacts (64) are for carrying low voltages of approximately 1 KV or less.

### Example 16

The electrical connector assembly (10) of any one of Example 1 to Example 15, additionally comprising: a second connector (22) including openings corresponding to and for receiving the pins (70, 72, 74) of each of the first electrical contacts, the second electrical contacts, and the third electrical contacts, for electrically coupling with the first connector (20).

### Example 17

A method for manufacturing an electrical connector (20) comprising: providing: a base (44); a first platform (50) extending longitudinally from the base (44) and extending transversely to a periphery (50a); and, a second platform (52) extending longitudinally from the first platform (50) and extending transversely to a periphery (52a), the periphery (52a) of the second platform (52) within the periphery (50a) of the first platform (50). The method is such that a plurality of first electrical contacts (60) are distributed along the periphery (50a) of the first platform (50); and, a plurality of second electrical contacts (62) are distributed along the periphery (52a) of the second platform (52).

### Example 18

The method of Example 17, wherein the providing additionally comprises providing a third platform (54) extending longitudinally from the second platform (52) and extending transversely to a periphery (54a), the periphery (54a) of the third platform (54) within the periphery (52a) of the second platform (52); and, distributing a plurality of third electrical contacts (64) along a transversely extending surface (54d) of the third platform (54).

### Example 19

The method of Example 17 or 18, wherein the distributing the plurality of the first electrical contacts (60) along the periphery (50a) of the first platform (50) includes spacing each of the first electrical contacts (60) apart from each other at a first predetermined distance; and, the distributing the plurality of the second electrical contacts (62) along the periphery (52a) of the second platform (52) includes spacing each of the second electrical contacts (62) apart from each other at a second predetermined distance.

### Example 20

The method of any one of Example 17 to Example 19, wherein the first predetermined distance and the second predetermined distance are the same.

### Example 21

The method of any one of Example 17 to Example 20, wherein the first predetermined distance and the second predetermined distance are different.

### Example 22

The method of any one of Example 17 to Example 21, wherein the first electrical contacts (60) and the second electrical contacts (62) are for carrying high voltages of approximately at least 4 KV.

### Example 23

The method of any one of Example 17 to Example 23, wherein the third electrical contacts (64) are for carrying low voltages of approximately 1 KV or less.

It will thus be appreciated that the examples of the present disclosure are not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An electrical connector assembly comprising:
a first connector having a longitudinal axis extending between a proximal end and a distal end of the first connector, and a transverse axis extending substantially perpendicular to the longitudinal axis, the first connector comprising:
a base;
a first platform extending longitudinally from the base and extending transversely to a periphery;
a second platform extending longitudinally from the first platform and extending transversely to a periphery, the periphery of the second platform within the periphery of the first platform;
a plurality of first electrical contacts distributed along the periphery of the first platform; and
a plurality of second electrical contacts distributed along the periphery of the second platform.

2. The electrical connector assembly of claim 1, wherein the first connector additionally comprises: a third platform extending longitudinally from the second platform and extending transversely to a periphery, the periphery of the third platform within the periphery of the second platform, the third platform supporting a plurality of third electrical contacts.

3. The electrical connector assembly of claim 1, wherein:
the plurality of first electrical contacts distributed along the periphery of the first platform are spaced apart from each other at a first predetermined distance;
the plurality of second electrical contacts distributed along the periphery of the second platform are spaced apart from each other at a second predetermined distance; and
wherein the third electrical contacts comprise pins, extending from surface of third platform and for electrically communication with a flat printed circuit.

4. The electrical connector assembly of claim 1, wherein:
the base is substantially cylindrical and is circular in cross section along the transverse axis,
the first platform and the second platform are circular in a cross section along the transverse axis and are concentric, and,
1) the plurality of first electrical contacts are arranged in an outer ring, and
2) the plurality of second electrical contacts are arranged in an inner ring, with respect to the outer ring.

5. The electrical connector assembly of claim 1, wherein the first electrical contacts and the second electrical contacts comprise solder cups.

6. The electrical connector assembly of claim 1, additionally comprising: a plurality of pins in the base, each pin of the plurality of pins in electrical communication with a corresponding one of the first or second electrical contacts.

7. The electrical connector assembly of claim 6, wherein the pins corresponding to the first electrical contacts are arranged on the base as a first ring, and, the pins corresponding to the second electrical contacts are arranged on the base in a second ring, inside of the first ring.

8. The electrical connector assembly of claim 6, additionally comprising:
a plurality of pins supported by the base within the second ring, the each of the pins in electrical communication with a corresponding third electrical contact; and
a second connector including openings corresponding to and for receiving the pins of each of the first electrical contacts, the second electrical contacts and the third electrical contacts, for electrically coupling with the first connector.

9. The electrical connector assembly of claim 1, wherein the first electrical contacts and the second electrical contacts are for carrying high voltages of approximately at least 4 KV.

10. The electrical connector assembly of claim 1, wherein the third electrical contacts are for carrying low voltages of approximately 1 KV or less.
